# EUROPÄISCHE PATENTSCHRIFT

(11) **EP 1 958 933 B1**
(45) Veröffentlichungstag und Bekanntmachung des Hinweises auf die Patenterteilung: **02.11.2011**
(21) Anmeldenummer: 08150940.8
(22) Anmeldetag: 01.02.2008
(51) Int. Cl.: C07C 319/28, C07C 323/58, C12P 13/12

(54) **Verfahren zur Reinigung von L-Cystein**
Process for purifying L-cysteine
Procédé destiné au nettoyage de L-cystéine

(30) Priorität: 14.02.2007 DE 102007007333
(43) Veröffentlichungstag der Anmeldung: 20.08.2008
(73) Patentinhaber: Wacker Chemie AG, 81737 München (DE)
(72) Erfinder: Böhm, Andreas, 81829, München (DE)
(74) Vertreter: Potten, Holger

(56) Entgegenhaltungen:
- EP-A- 0 250 987
- EP-A- 1 571 223

## Beschreibung

Die Erfindung betrifft ein Verfahren zur Reinigung von L-Cystein aus einer L-Cystein-haltigen Fermenterbrühe.

L-Cystein ist eine Aminosäure, die aufgrund der guten Löslichkeit in Wasser und der hohen Empfindlichkeit der SH-Gruppe gegenüber einer Vielzahl von Reagenzien, z.B. gegenüber Oxidationsmitteln, nur sehr schwer und unter hohem Aufwand zu reinigen und zu isolieren ist. Die Aminosäure L-Cystin ist aufgrund der sehr geringen Wasserlöslichkeit und der vergleichsweise hohen Stabilität, z.B. gegenüber Oxidationsmitteln, dagegen leicht und in sehr hoher Reinheit auch aus komplexen SubstanzMischungen, wie z.B. Proteinhydrolysaten oder L-Cystin enthaltenden Fermenterbrühen, zu reinigen und zu isolieren. In DE10040176A1 ist beispielsweise ein sehr einfaches Verfahren zur Isolierung von L-Cystin aus zellhaltigen Suspensionen bzw. Fermenterbrühen beschrieben.

Die technische Herstellung von L-Cystein aus komplexen Substanzmischungen, wie z.B. Proteinhydrolysaten (z.B. aus menschlichen Haaren oder tierischen Quellen (z.B. Federn oder Borsten)) oder entsprechenden Fermenterbrühen, erfolgt deshalb zunächst durch Isolierung des schwerlöslichen L-Cystins in gereinigter Form. In solchen komplexen Substanzmischungen evtl. vorhandenes L-Cystein oder andere L-Cystein-Derivate werden dabei gezielt und möglichst vollständig, z.B. durch Oxidation, in L-Cystin überführt. L-Cystin wird dann durch anschließende Reduktion (z.B. durch Elektrolyse) zu L-Cystein umgesetzt. Dieses Verfahren ist jedoch mit Nachteilen behaftet, da L-Cystein in einem zweistufigen Verfahren über die Zwischenstufe L-Cystin aufwändig hergestellt werden muss.

Die direkte Isolierung von L-Cystein aus einer Lösung, welche L-Cystein, L-Cystin, L-Serin und ein anorganisches Salz enthält, ist in EP0250987B1 beschrieben. Durch Zugabe von Chlorwasserstoff wird zunächst bei mindestens 20°C L-Cystin und das anorganische Salz kristallisiert und abfiltriert. Aus der verbleibenden Lösung, welche noch L-Cystein und L-Serin enthält, wird dann bei maximal 10°C L-Cystein-Hydrochlorid-Monohydrat in hoher Reinheit kristallisiert und isoliert. Das Verfahren ist jedoch auf Lösungen, welche L-Cystein, L-Cystin, L-Serin und ein anorganisches Salz enthalten, beschränkt, es ermöglicht nicht die Gewinnung von L-Cystein in hoher Ausbeute und Reinheit aus komplexen Substanzmischungen, wie Proteinhydrolysaten oder Fermentationsbrühen.

In EP1645623A1, EP1298200B1, US20050221453A1, EP1234874A1 und EP1571223A2 ist die Isolierung von L-Cystein aus Fermentationsbrühen durch eine Kombination aus Ionenaustausch, Kristallisation und anderen bekannten Methoden beschrieben. Es sind jedoch keine Angaben zur konkreten Vorgehensweise, zu den erzielten Ausbeuten und Reinheiten dokumentiert.

In EP1650296A1 ist die Isolierung von L-Cystein aus Fermentationsbrühen durch Entfernung der Feststoffe durch Zentrifugation oder Membranfiltration und anschließende Isolierung und Reinigung der Aminosäure durch Ionenaustausch, Aufkonzentration und Kristallisation beschrieben. Auch hier finden sich keine Angaben zur konkreten Vorgehensweise, zu den erzielten Ausbeuten und Reinheiten.

Die direkte und kostengünstige Reinigung und Herstellung von L-Cystein aus komplexen Substanzmischungen, wie z.B. L-Cystein enthaltenden Fermenterbrühen eines Mikroorganismus, ist also nach wie vor ein ungelöstes Problem. Es ist kein im technischen Maßstab umsetzbares Verfahren bekannt, wonach L-Cystein aus L-Cystein enthaltenden Fermenterbrühen kostengünstig, direkt und ohne Derivatisierung, z.B. durch Oxidation zu L-Cystin mit anschließender Reduktion zu L-Cystein, in hoher Reinheit und/oder Ausbeute gewonnen werden kann.

In EP0885962B1, EP0858510B1 und EP1220940B1 sind Verfahren zur fermentativen Herstellung von L-Cystein beschrieben. Diese Verfahren ermöglichen einen kostengünstigen Zugang zu Fermentationsbrühen, die L-Cystein in großer Menge enthalten.

Eine derartige L-Cystein enthaltende Fermenterbrühe ist ein äußerst komplexes Substanzgemisch. Sie enthält neben L-Cystein in der Regel L-Cystin, welches unter den Bedingungen der Fermentation, insbesondere durch Oxidation mit vorhandenem Sauerstoff, leicht aus L-Cystein gebildet wird. Bei Anwesenheit von Aldehyden oder Ketonen können des Weiteren entsprechende Hemithioketale und/oder Thiazolidinderivate von L-Cystein, wie z.B. in EP0885962B1 beschrieben, vorliegen. In geringen Mengen können die Fermenterbrühen auch weitere Aminosäuren oder deren Derivate enthalten. Sie enthalten ferner in der Regel Kohlenhydrate, Salze organischer und anorganischer Kationen und Anionen, z.B. Alkali- und Erdalkalimetallsalze sowie Spuren an Schwermetallsalzen (z.B. Fe, Cu, Mn, Zn etc.), Farbstoffe und weitere Verunreinigungen und Zusätze, wie z.B. unerwünschte Stoffwechselprodukte der bei der Fermentation eingesetzten Mikroorganismen. Ferner können die Fermenterbrühen, wie z.B. in EP0885962B1, EP0858510B1 und EP1220940B1 beschrieben, noch die in der Fermentation eingesetzten Roh- und Inhaltsstoffe enthalten, z.B. übliche C-Quellen, wie Glucose, Lactose, Stärke und dergleichen, N-Quellen, wie Ammoniak/Ammonium oder Proteine oder Proteinhydrolysate und dergleichen, sowie S-Quellen, wie beispielsweise Sulfid, Sulfit, Sulfat, Thiosulfat oder Dithionit und dergleichen. Da L-Cystein eine schwefelhaltige Aminosäure ist, wird während der Fermentation in aller Regel eine S-Quelle, wie beispielsweise Sulfid, Sulfit, Sulfat, Thiosulfat oder Dithionit zugefüttert, um den für die Bildung von L-Cystein benötigten Schwefel in ausreichender Menge bereitzustellen. Weiterhin ist in den Fermenterbrühen durch den während der Fermentation eingetragenen Sauerstoff auch gelöster Sauerstoff enthalten. Der pH-Wert dieser Fermenterbrühen liegt üblicherweise, wie z.B. in EP0885962B1 beschrieben, bei pH 7.

Die Oxidation von L-Cystein, z.B. zu L-Cystin, in einer Fermenterbrühe oder einer sonstigen Lösung kann durch alle Oxidationsmittel erfolgen, welche SH-Gruppen zu oxidieren vermögen. Neben der Bildung von L-Cystin als primärem Produkt der Oxidation von L-Cystein können dabei auch höher oxidierte Verbindungen des L-Cysteins und/oder L-Cystins entstehen. Ein unmittelbarer Ausbeuteverlust an L-Cystein ist somit bei Anwesenheit solcher Oxidationsmittel in L-Cystein enthaltenden Fermenterbrühen bzw. L-Cystein enthaltenden Lösungen die Folge.

Oxidationsmittel, welche SH-Gruppen (und somit auch L-Cystein) bei pH-Werten von pH<5 zu oxidieren vermögen sind z.B. Sauerstoff sowie Schwefel-Sauerstoffverbindungen. Diese Oxidationsmittel sind in L-Cystein enthaltenden Fermenterbrühen in der Regel in variablen Mengen enthalten. Schwefel-Sauerstoffverbindungen, wie z.B. Thiosulfat, werden hierbei entweder, wie z.B. in EP0885962B1 beschrieben, direkt als S-Quelle während der Fermentation zugesetzt oder können aus anderen zugesetzten S-Quellen, wie beispielsweise Sulfid, Sulfit, Sulfat, Thiosulfat oder Dithionit und dergleichen, während der Fermentation entstehen. So kann z.B. durch Sauerstoff, welcher während der Fermentation eingetragen wird, Sulfid bzw. Schwefelwasserstoff u.a. leicht zu Thiosulfat oxidiert werden. Die komplexe Chemie der Schwefel-Sauerstoffverbindungen bzw. deren Bildung ist z.B. auch in Hollemann-Wiberg, Lehrbuch der Anorganischen Chemie, 91.-100. Auflage, Walter de Gruyter, Berlin - New York, 1985, S. 485-523 ausführlich beschrieben worden.

L-Cystein wird durch Sauerstoff als Oxidationsmittel bevorzugt bei hohen pH-Werten besonders leicht oxidiert. Eine L-Cystein-Lösung kann gegenüber Oxidation mit Sauerstoff dadurch stabilisiert werden, dass der pH-Wert der Lösung gesenkt wird. So ist ferner bekannt, dass wässrige Lösungen von L-Cystein-Hydrochlorid, bevorzugt in Salzsäure, wesentlich stabiler gegenüber Oxidation mit Sauerstoff sind als z.B. wässrige Lösungen von L-Cystein mit pH-Werten von pH 7 oder höher.

Im Gegensatz dazu nimmt bei einer Vielzahl von Schwefel-Sauerstoffverbindungen die Oxidationskraft gegenüber SH-Gruppen, und somit auch gegenüber L-Cystein, mit sinkendem pH-Wert zum Teil deutlich zu. Diese Oxidationsmittel können in L-Cystein enthaltenden Fermenterbrühen oder auch daraus aufgereinigten L-Cystein enthaltenden Lösungen enthalten sein und bei pH-Werten, welche z.B. während der Fermentation auftreten, L-Cystein nicht oder nur in sehr geringem Maße oxidieren, dagegen mit sinkendem pH-Wert, besonders bei pH-Werten von pH<5, L-Cystein teilweise sehr gut oxidieren.

Beispiele für Schwefel-Sauerstoff-Verbindungen, welche SH-Gruppen und somit auch L-Cystein zu oxidieren vermögen, sind z.B. Schwefeldioxid oder Schwefeltrioxid. Auch beim Ansäuern von Sulfit-Lösungen wird sukzessive Schwefeldioxid freigesetzt, welches dann SH-Gruppen zu oxidieren vermag. Auch Thiosulfat, welches als Schwefel-Quelle in der Fermentation von L-Cystein bevorzugt eingesetzt wird und bei einem pH-Wert pH>5, bevorzugt pH = 7, wie er während der Fermentation auftritt, L-Cystein praktisch nicht oxidiert, oxidiert bei einem pH-Wert <5 L-Cystein, wobei mit sinkendem pH-Wert die Oxidationskraft bzw. die Geschwindigkeit der Oxidation zunimmt.

Eine Reihe weiterer Schwefel-Sauerstoff-Verbindungen und ihre Fähigkeit zur Oxidation von SH-Gruppen bevorzugt im sauren Milieu ist auch in Hollemann-Wiberg, Lehrbuch der Anorganischen Chemie, 91.-100. Auflage, Walter de Gruyter, Berlin - New Y-ork, 1985, S. 485-523 beschrieben.

Des Weiteren gibt es eine Reihe von Verbindungen, welche die Oxidation von SH-Gruppen katalysieren können. So ist z.B. bekannt, dass Schwermetallsalze die Oxidation von Cystein zu Cystin effektiv katalysieren können. Schwermetallsalze, wie z.B. Eisen- oder Zinksalze, sind auch häufig essentielle Zusätze bei Fermentationen und somit auch in den entsprechenden Fermentationsbrühen in üblicherweise geringen Mengen enthalten.

Enthält eine L-Cystein enthaltende Lösung bzw. Fermenterbrühe ein Oxidationsmittel, welches bei pH-Werten von pH<5 L-Cystein, z.B. zu L-Cystin, zu oxidieren vermag, so hat dies einen unmittelbaren Ausbeuteverlust zur Folge, falls solche Lösungen bzw. Fermenterbrühen auf pH<5 angesäuert werden. Da das bevorzugte Endprodukt L-Cystein-Hydrochlorid-Monohydrat aus stark sauren Lösungen kristallisiert wird, ist in Abhängigkeit vom Gehalt des Oxidationsmittels bezogen auf L-Cystein ein Ausbeuteverlust von bis zu 100% des vorhandenen L-Cysteins möglich.

Aufgabe der vorliegenden Erfindung ist es, ein einfaches, kostengünstiges und technisch durchführbares Verfahren zur Herstellung einer gereinigten L-Cystein enthaltenden Lösung, aus einer L-Cystein haltigen Fermenterbrühe bereitzustellen. Aus diesen Lösungen kann dann bei Bedarf L-Cystein, L-Cystein-Hydrochlorid oder L-Cystein-Hydrochlorid-Monohydrat als Feststoff, z.B. durch Kristallisation, gewonnen werden.

Diese Aufgabe wird gelöst durch ein Verfahren bei dem eine L-Cystein haltige Fermenterbrühe, die das Oxidationsmittel Thiosulfat umfasst, bei einem pH Wert von pH 5 bis 9 mit einem stark sauren Ionenaustauscher in der H⁺-Form in Kontakt gebracht wird, wobei ein pH-Wert von pH<5, bevorzugt pH<2 in der Fermenterbrühe auftritt und wobei das L-Cystein an den Ionenaustauscher bindet und das gebundene L-Cystein mittels eines Elutionsmittels von dem Ionenaustauscher entfernt wird.

Eine L-Cystein enthaltende Fermenterbrühe lässt sich wie bereits beschrieben erhalten. Diese Fermenterbrühe wird, vorzugsweise nach Abtrennung der Zellen und Feststoffe, mit Hilfe eines Ionenaustauschers aufgereinigt.

Dies kann z.B. **dadurch geschehen, dass** die Fermenterbrühe über eine mit einem stark sauren Kationenaustauscher gepackte Säule gepumpt wird.

Der pH-Wert der applizierten Fermenterbrühe liegt dabei bei einem pH-Wert von 5-9 und bevorzugt bei pH = 5-7, da es sonst, in Abhängigkeit von der Menge des Oxidationsmittels, zu einem deutlichen Ausbeuteverlust durch Oxidation von L-Cystein kommen kann.

Zum Vergleich dazu kann eine L-Cystein enthaltende Lösung oder Fermenterbrühe, welche nur sehr geringe Mengen oder überhaupt kein entsprechendes Oxidationsmittel enthält, prinzipiell bei jedem pH-Wert von 1-14 appliziert werden. Bei Verwendung eines stark sauren Kationenaustauschers wird dann die Lösung sogar bevorzugt bei pH-Werten von 1-5 appliziert, da bei diesen pH-Werten die Bindung von L-Cystein an den Ionenaustauscher besonders effektiv ist und bei Abwesenheit eines störenden Oxidationsmittels auch keine Oxidation und somit auch kein Verlust von L-Cystein auftritt.

Wird eine L-Cystein enthaltende Lösung bzw. Fermenterbrühe mit dem stark sauren Kationenaustauscher in der H⁺-Form, in Kontakt gebracht, so wird bei diesem Vorgang L-Cystein nahezu quantitativ an den Kationenaustauscher gebunden. Auch andere Aminosäuren, z.B. L-Cystin, und/oder weitere Kationen, welche in der Fermenterbrühe enthalten sein können, können hierbei an das Harz binden. Durch geschickte Wahl der Adsorptionsbedingungen (Flussrate, Beladung, Konzentration, Temperatur, Harz etc.) kann die Bindung dieser Verunreinigungen aber zum Teil deutlich minimiert werden. Eine Vielzahl von weiteren Verunreinigungen, wie z.B. Neutralverbindungen oder Anionen bzw. deren korrespondierende Säuren, bindet nicht an das Harz und befindet sich im Eluat. Auch anionische Oxidationsmittel bzw. deren korrespondierende Säuren sowie auch die Zersetzungsprodukte dieser Oxidationsmittel binden nicht an den Kationenaustauscher. Eine wirkungsvolle Abtrennung dieser Verunreinigungen kann bei diesem Verfahrensschritt somit erreicht werden.

Wird der stark saure Kationenaustauscher in der H⁺-Form eingesetzt, so kommt es durch den Ionenaustausch-Prozess direkt im Harz zu einem starken pH-Shift der das Harz durchfließenden Lösung. Der pH-Wert der an dem Austauschprozess beteiligten L-Cystein enthaltenden Lösung bzw. Fermenterbrühe sowie des den Kationenaustauscher verlassenden Eluats liegt deshalb üblicherweise bei pH<2 und häufig sogar bei pH<1 (siehe Beispiele).

Das an dem Ionenaustauscher gebundene L-Cystein wird anschließend mit gängigen Säuren oder Basen oder Salzlösungen vom Harz eluiert. Als Säuren werden bevorzugt starke Säuren und besonders bevorzugt Salzsäure eingesetzt. Als Basen werden bevorzugt Amine und besonders bevorzugt Ammoniak eingesetzt. Insbesondere geeignet sind wässrige Salzsäure eine wässrige Salzlösung, ein Amin, eine Base oder eine starke Base.

Bei Elution mit Ammoniak wird durch den mit L-Cystein beladenen Ionenaustauscher wässriger Ammoniak unterschiedlicher Normalitäten, bevorzugt 0,1-12N Ammoniak und besonders bevorzugt 1-2N Ammoniak, gepumpt. Die erhaltene ammoniakalische gereinigte Lösung von L-Cystein kann anschließend z.B. optional mit Aktivkohle entfärbt und aufkonzentriert werden. Aus den konzentrierten Lösungen kann L-Cystein dann z.B. in hoher Reinheit und Ausbeute kristallisiert oder mit einem geeigneten Fällungsmittel gefällt werden.

Bei Elution mit Salzsäure wird durch den mit L-Cystein beladenen Ionenaustauscher wässrige Salzsäure unterschiedlicher Normalitäten, bevorzugt 0,1-12N HCl und besonders bevorzugt 1-2N HCl, gepumpt.

Das L-Cystein enthaltende saure Eluat enthält kein Oxidationsmittel mehr, welches bei pH-Werten von pH<5 L-Cystein zu oxidieren vermag. Daher sind diese Lösungen, wie im Stand der Technik beschrieben, auch stabil gegenüber Oxidation von L-Cystein, z.B. durch Sauerstoff. Das Oxidationsmittel, welches bei pH-Werten von pH<5 L-Cystein zu oxidieren vermag, wird entweder bei der Adsorption von L-Cystein an den Ionenaustauscher abgetrennt oder vernichtet, oder auf eine andere Art und Weise bei der Elution von L-Cystein mit Säure abgetrennt oder vernichtet.

Bei Elution des L-Cysteins mit Salzsäure wird eine gereinigte HCl-Lösung von L-Cystein erhalten. Diese Lösung kann optional aufkonzentriert und z.B. mit Aktivkohle entfärbt werden. Ggf. nach Zugabe von HCl wird das technisch besonders wichtige Produkt L-Cystein-Hydrochlorid-Monohydrat kristallisiert.

Durch Fraktionierung des Eluats und/oder Anwendung eines Gradienten kann L-Cystein mit einer signifikant höheren Reinheit als in der ursprünglich applizierten Fermenterbrühe vorhanden, erhalten werden. Durch geschickte Wahl der Elutionsbedingungen ist es z.B. möglich, L-Cystein (Elution bevorzugt mit 1N HCl) von L-Cystin (Elution bevorzugt mit 2N HCl) auf der Säule voneinander zu trennen (siehe auch Beispiele 7 und 9). Diese Verfahrensführung ist besonders vorteilhaft, da L-Cystin durch andere Methoden, wie z.B. bei der Kristallisation von L-Cystein-Hydrochlorid-Monohydrat, nur sehr schwer von L-Cystein-Hydrochlorid-Monohydrat abgetrennt werden kann.

Überraschend zeigte sich nun, dass das L-Cystein aus einer Lösung, die als Oxidationsmittel Thiosulfat enthält, welches bei pH-Werten <5 L-Cystein zu oxidieren vermag, ohne nennenswerte Oxidation an einem stark sauren Kationenaustauscher in der H⁺-Form, adsorbiert und wieder eluiert werden kann, auch wenn bei diesen Verfahrensschritten pH-Werte von pH<5, bevorzugt pH<2 in der L-Cystein enthaltenden Fermenterbrühe auftritt.

Dies ist besonders überraschend, da beim Ansäuern einer L-Cystein enthaltenden Fermenterbrühe, welche ein Oxidationsmittel enthält, welches bei pH-Werten <5 L-Cystein zu oxidieren vermag, mit gängigen Säuren, wie z.B. Salzsäure oder Schwefelsäure, auf pH<5, bevorzugt pH<2, wie erwartet eine deutliche Oxidation von L-Cystein zu L-Cystin beobachtet wird, womit ein unmittelbarer Ausbeuteverlust verbunden ist (siehe Beispiele 3 und 5). Dies übt auf die Wirtschaftlichkeit des Verfahrens einen entscheidenden negativen Einfluss aus.

Die nach dem erfindungsgemäßen Verfahren erhaltene L-Cystein enthaltende Lösung kann durch Destillation aufkonzentriert oder mittels Aktivkohle entfärbt werden. Es ist auch möglich, aus diesen Lösungen L-Cystein oder L-Cystein-Hydrochlorid-Monohydrat zu kristallisieren und somit einen weiteren Reinigungseffekt zu erzielen.

Aus der erfindungsgemäß erhaltenen Lösung von L-Cystein wird bevorzugt L-Cystein-Hydrochlorid-Monohydrat kristallisiert.

Dies geschieht durch Aufkonzentrieren der erfindungsgemäß erhaltenen Lösung. Aus diesen konzentrierten Lösungen wird, ggf. nach Zugabe von Salzsäure und ggf. nach Abkühlen auf bis zu -20°C, bevorzugt L-Cystein-Hydrochlorid-Monohydrat kristallisiert. Enthält die L-Cystein-Lösung neben L-Cystein noch Metall- und/oder Ammoniumsalze oder z.B. L-Cystin, so können diese Verbindungen z.B. durch fraktionierte Kristallisation abgetrennt werden. Dies kann z.B. in Anlehnung an das in EP 0250987B1 beschriebene Verfahren geschehen, indem bei mindestens 20°C durch Zugabe von Salzsäure die entsprechenden Metall- und/oder Ammoniumchloride und L-Cystin kristallisiert und abfiltriert werden und danach aus den erhaltenen HCl-Lösungen von L-Cystein durch Abkühlen auf bis zu -20°C L-Cystein-Hydrochlorid-Monohydrat kristallisiert, isoliert und getrocknet wird.

Mit dem beschriebenen Verfahren ist es möglich L-Cystein effektiv, mit guten Ausbeuten und wirtschaftlich aus einer L-Cystein enthaltenden Fermenterbrühen aufzureinigen. Wenn es erforderlich oder gewünscht ist, können unter bestimmten Verfahrensbedingungen z.B. auch evtl. vorhandene Derivate von L-Cystein, z.B. L-Cystin oder Thiazolidin-Derivate, zu L-Cystein umgewandelt werden und somit die erzielte Ausbeute erhöhen. So ist z.B. die Spaltung von Thiazolidin-Derivaten des Cysteins an stark sauren Kationenaustauschern bekannt und in EP1059288B1 beschrieben. Die Spaltung von L-Cystin zu L-Cystein durch Zugabe von geeigneten Reduktionsmitteln ist ebenfalls denkbar.

Mit dem beschriebenen Verfahren ist es möglich, den Fremdaminosäure-Gehalt auf <5%, bevorzugt <1% bezogen auf L-Cystein zu senken. Des Weiteren ist es auch möglich, den Salzgehalt bezogen auf L-Cystein auf <10%, bevorzugt <1% zu senken. Außerdem ist es mit dem beschriebenen Verfahren möglich, L-Cystein bzw. L-Cystein-Hydrochlorid oder L-Cystein-Hydrochlorid-Monohydrat in einer Reinheit von >98% und einer optischen Reinheit von >99% aus L-Cystein enthaltenden Fermenterbrühen herzustellen. Bevorzugt erfolgt vor Durchführung des erfindungsgemäßen Verfahrens in einem ersten Verfahrensschritt die Abtrennung von Mikroorganismenzellen und/oder unlöslichen Bestandteilen aus der L-Cystein enthaltenden Fermenterbrühe. Dies geschieht beispielsweise durch Zentrifugation, Filtration, Dekantieren, Membranfiltration oder eine andere dem Fachmann geläufige Methode zur Abtrennung von Zellen/Feststoffen aus einer Fermenterbrühe. Die Abtrennung erfolgt ggf. unter Zusatz eines Filterhilfsmittels, wie z.B. Celite®, Aktivkohle oder Diatomeenerde. Bei diesem Verfahrensschritt werden vorteilhafterweise neben den Zellen weitere unlösliche Bestandteile, wie z.B. aus der Lösung ausgefallenes Cystin oder Niederschläge anderer schwerlöslicher Bestandteile, die bei der Fermentation des Mikroorganismus entstehen können, ebenfalls von der Fermenterbrühe abgetrennt. Des Weiteren können bei diesem Verfahrensschritt z.B. auch Makromoleküle, wie z.B. Proteine, abgetrennt bzw. an ein ggf. eingesetztes Filterhilfsmittel oder Aktivkohle oder Ähnlichem adsorbiert und somit abgetrennt werden. Die mittels dieser Vorbehandlung erhaltene L-Cysteinhaltige Lösung fällt im Sinne der vorliegenden Erfindung auch unter den Begriff der Fermenterbrühe.

Die folgenden Beispiele dienen der weiteren Erläuterung der Erfindung.

### Beispiel 1: Stabilität einer O₂-haltigen Lösung von L-Cystein (pH 1)

100 ml einer Lösung von L-Cystein (c = 18g/l) in sauerstoffhaltigem Wasser werden mit 20% HCl tropfenweise auf pH = 1 angesäuert. Nach 5 Minuten Rühren bei RT weist die Lösung einen L-Cystein-Gehalt von 18g/l und einen Gehalt von <0,1g/l an gelöstem L-Cystin auf. Die Lösung ist bzgl. des L-Cystein-Gehalts über mehr als 24h stabil.

### Beispiel 2: Stabilität einer O₂-haltigen sauren Lösung von L-Cystein (pH 5)

100 ml einer Lösung von L-Cystein (c = 18g/l) in sauerstoffhaltigem Wasser werden mit 20% HCl tropfenweise auf pH = 5 angesäuert. Nach 5 Minuten Rühren bei RT weist die Lösung einen L-Cystein-Gehalt von 18g/l und einen Gehalt von <0,1g/l an gelöstem L-Cystin auf. Nach 24h Rühren bei RT weist die Lösung einen L-Cystein-Gehalt von 17,6 g/l und einen L-Cystin-Gehalt von 0,4g/l auf.

### Beispiel 3: Stabilität einer thiosulfat-haltigen sauren Lösung von L-Cystein (pH 1)

100 ml einer Lösung von L-Cystein (c = 18g/l) und Ammoniumthiosulfat (c = 2,5g/l Thiosulfat) in Wasser werden mit 20% HCl tropfenweise auf pH = 1 angesäuert. Nach 5 Minuten Rühren bei RT weist die getrübte Lösung einen L-Cystein-Gehalt von 12g/l, einen Thiosulfat-Gehalt von <0,2g/l und einen Gehalt von 6g/l an gelöstem L-Cystin auf. Nach 24h Rühren bei RT ist der L-Cystein-Gehalt auf <5g/l abgesunken.

### Beispiel 4: Stabilität einer thiosulfat-haltigen sauren Lösung von L-Cystein (pH 5)

100 ml einer Lösung von L-Cystein (c = 18g/l) und Ammoniumthiosulfat (c = 2,5g/l Thiosulfat) in Wasser werden mit 20% HCl tropfenweise auf pH = 5 angesäuert. Nach 5 Minuten Rühren bei RT weist die Lösung einen L-Cystein-Gehalt von 17,5g/l, einen Thiosulfat-Gehalt von 2,4g/l und einen Gehalt von <0,5g/l an gelöstem L-Cystin auf. Nach 24h Rühren bei RT ist der L-Cystein-Gehalt auf 16,0 g/l abgesunken.

### Beispiel 5: Stabilität einer thiosulfat-haltigen sauren Fermenterbrühe von L-Cystein (pH 1)

100 ml einer Mikroorganismen enthaltenden Fermenterbrühe mit einem pH-Wert von pH = 7, einem L-Cystein-Gehalt von 18g/l, einem Thiosulfat-Gehalt von 2,5g/l und einem Gehalt von 1,1g/l an gelöstem L-Cystin werden mit 20% Salzsäure tropfenweise auf pH = 1 angesäuert und zur Abtrennung der Biomasse und von Feststoffen 20 Minuten zentrifugiert (8000 rpm / min). Die resultierende klare Lösung weist einen L-Cystein-Gehalt von 12g/l, einen Thiosulfat-Gehalt von <0,2g/l und einen Gehalt von 7g/l an gelöstem L-Cystin auf.

### Beispiel 6: Stabilität einer thiosulfat-haltigen sauren Fermenterbrühe von L-Cystein (pH 5)

100 ml einer Mikroorganismen enthaltenden Fermenterbrühe mit einem pH-Wert von pH = 7, einem L-Cystein-Gehalt von 18g/l, einem Thiosulfat-Gehalt von 2,5g/l und einem Gehalt von 1,1g/l an gelöstem L-Cystin werden mit 20% Salzsäure tropfenweise auf pH = 5 angesäuert und zur Abtrennung der Biomasse und von Feststoffen 20 Minuten zentrifugiert (8000 rpm / min). Die resultierende klare Lösung weist einen L-Cystein-Gehalt von 17,3 g/l, einen Thiosulfat-Gehalt von 2,3g/l und einen Gehalt von 1,1g/l an gelöstem L-Cystin auf.

### Beispiel 7: Aufreinigung einer thiosulfathaltigen Fermenterbrühe mittels des erfindungsgemäßen Verfahrens

1000 ml einer Mikroorganismen enthaltenden Fermenterbrühe mit einem pH-Wert von pH = 7, einem L-Cystein-Gehalt von 18g/l, einem Thiosulfat-Gehalt von 2,5g/l und einem Gehalt von 1,1g/l an gelöstem L-Cystin werden mit 20% Salzsäure tropfenweise auf pH = 5 angesäuert und zur Abtrennung der Biomasse und von Feststoffen 20 Minuten zentrifugiert (8000 rpm / min). Die resultierende klare Lösung wird über eine Kationenaustauschersäule (200ml Amberlite IR 120H, stark sauer, H⁺-Form) gepumpt. Hierbei werden L-Cystein und L-Cystin nahezu quantitativ an das Harz gebunden und gegen Protonen ausgetauscht. Ggf. werden auch weitere Aminosäuren sowie in der Lösung vorhandene Kationen an das Harz gebunden. Während dieses Austauschprozesses tritt in der das Harz durchströmenden Lösung ein massiver pH-Shift zu niedrigen pH-Werten auf. Der pH-Wert des Eluats liegt im Bereich von pH = 0-1. Des Weiteren enthält das Eluat eine Reihe von Verunreinigungen, welche nicht an das Harz binden (z.B. Neutral-Verbindungen, Anionen bzw. deren korrespondierende Säuren etc.), jedoch keine oder nur geringe Mengen an Aminosäuren. Durch das stark saure Milieu wird Thiosulfat vollständig zersetzt und ist im Eluat nicht mehr nachweisbar. Aufgrund der Zersetzung von Thiosulfat im sauren Milieu kann das getrübte Eluat u.a. kolloidalen Schwefel enthalten.

Nach Waschen der Ionenaustauscher-Säule mit 400 ml Wasser wird an das Harz gebundenes L-Cystein mit 1000 ml 1N HCl eluiert. Die Produktfraktionen enthalten mehr als 90% des in der applizierten Fermenterbrühe vorhandenen L-Cysteins und nur sehr geringen Mengen an L-Cystin (0,3g; <2% bezogen auf L-Cystein). Mit 2N HCl kann an der Ionenaustauscher-Säule verbliebenes, gebundenes L-Cystin eluiert werden (<2g).

### Beispiel 8: Vergleichsbeispiel: Aufreinigung einer thiosulfathaltigen Fermenterbrühe / Adsorption bei pH = 1

1000 ml einer Mikroorganismen enthaltenden Fermenterbrühe mit einem pH-Wert von pH = 7, einem L-Cystein-Gehalt von 18g/l, einem Thiosulfat-Gehalt von 2,5g/l und einem Gehalt von 1,1g/l an gelöstem L-Cystin werden mit 20% Salzsäure tropfenweise auf pH = 1 angesäuert wird und zur Abtrennung der Biomasse und von Feststoffen 20 Minuten zentrifugiert (8000 rpm / min). Die resultierende Lösung wird über eine Kationenaustauschersäule (200ml Amberlite IR 120H, stark sauer, H⁺-Form) gepumpt. Hierbei werden L-Cystein und L-Cystin nahezu quantitativ an das Harz gebunden und gegen Protonen ausgetauscht. Ggf. werden auch weitere Aminosäuren sowie in der Lösung vorhandene Kationen an das Harz gebunden. Während dieses Austauschprozesses tritt in der das Harz durchströmenden Lösung ein weiterer pH-Shift zu niedrigen pH-Werten auf. Der pH-Wert des Eluats liegt im Bereich von pH = 0-1. Des Weiteren enthält das Eluat eine Reihe von Verunreinigungen, welche nicht an das Harz binden (z.B. Neutral-Verbindungen, Anionen bzw. deren korrespondierende Säuren etc.), jedoch keine oder nur geringe Mengen an Aminosäuren. Thiosulfat ist im Eluat nicht mehr nachweisbar. Aufgrund der Zersetzung von Thiosulfat im sauren Milieu kann das getrübte Eluat u.a. kolloidalen Schwefel enthalten. Nach Waschen der Ionenaustauscher-Säule mit 400 ml Wasser wird an das Harz gebundenes L-Cystein mit 1000 ml 1N HCl eluiert. Die Produktfraktionen enthalten weniger als 60% des in der Fermenterbrühe vorhandenen L-Cysteins.

### Beispiel 9: Vergleichsbeispiel: Aufreinigung einer thiosulfatarmen/freien Fermenterbrühe

1000 ml einer Mikroorganismen enthaltenden Fermenterbrühe mit einem pH-Wert von pH = 7, einem L-Cystein-Gehalt von 18g/l, einem Thiosulfat-Gehalt von <0,1g/l, bevorzugt 0g/l und einem Gehalt von 1,1g/l an gelöstem L-Cystin werden mit 20% Salzsäure tropfenweise auf pH = 1-5, bevorzugt pH = 3 angesäuert und zur Abtrennung der Biomasse und von Feststoffen 20 Minuten zentrifugiert (8000 rpm / min). Die resultierende klare Lösung (Cystein-Gehalt: 17,8g/l) wird über eine Kationenaustauschersäule (200ml Amberlite IR 120H, stark sauer, H⁺-Form) gepumpt. Hierbei werden L-Cystein, L-Cystin, ggf. weitere Aminosäuren sowie in der Lösung vorhandene Kationen nahezu quantitativ an das Harz gebunden und gegen Protonen ausgetauscht. Während dieses Austauschprozesses tritt in der das Harz durchströmenden Lösung ein massiver pH-Shift zu niedrigen pH-Werten auf. Der pH-Wert des Eluats liegt im Bereich von pH = 0-1. Des Weiteren enthält das Eluat eine Reihe von Verunreinigungen, welche nicht an das Harz binden (z.B. Neutral-Verbindungen, Anionen bzw. deren korrespondierende Säuren etc.), jedoch keine oder nur geringe Mengen an Aminosäuren. Aufgrund der sehr geringen bzw. nicht vorhandenen Thiosulfat-Mengen in der eingesetzten Fermenterbrühe (<0,1g/l) enthält das klare Eluat keinen kolloidalen Schwefel.

Nach Waschen der Ionenaustauscher-Säule mit 400 ml Wasser wird an das Harz gebundenes L-Cystein mit 1000 ml 1N HCl eluiert. Alternativ kann L-Cystein auch mit wässrigem Ammoniak eluiert werden. Die Produktfraktionen enthalten mehr als 90% des in der applizierten Fermenterbrühe vorhandenen L-Cysteins und nur sehr geringe Mengen an L-Cystin (0,3g; <2% bezogen auf L-Cystein). Mit 2N HCl kann an der Ionenaustauscher-Säule verbliebenes, gebundenes L-Cystin eluiert werden (<2g/l).

### Beispiel 10: Aufreinigen der HCl-Eluate aus Bsp. 7 und 9 zu L-Cystein-Hydrochlorid-Monohydrat

500 ml einer gereinigten L-Cystein enthaltenden Lösung (Eluat mit 1N HCl) aus Beispiel 7 oder 9 (L-Cystein-Gehalt = 17g/l) werden auf 25ml aufkonzentriert. Nach Zugabe von konzentrierter HCl oder durch Einleiten von HCl-Gas werden ggf. bei 20-60°C bevorzugt Chloride anorganischer Alkali- und Erdalkalimetallionen sowie Ammoniumchlorid kristallisiert. Nach Filtration wird die L-Cystein enthaltende Mutterlauge auf -10°C gekühlt und L-Cystein-Hydrochlorid-Monohydrat kristallisiert. Je nach Reinheit der eingesetzten L-Cystein enthaltenden Lösung kann L-Cystein-Hydrochlorid-Monohydrat in Ausbeuten von >80% und Reinheiten von >90% aus diesen Lösungen in kristalliner Form erhalten werden. Durch geeignete Steuerung der Kristallisationsbedingungen, fraktionierte Kristallisation und/oder Umkristallisation können Reinheiten bis hinauf zu >98% erzielt werden.

## Patentansprüche

1. Verfahren zur Reinigung einer L-Cystein haltigen Fermenterbrühe, **dadurch gekennzeichnet, dass** die Fermenterbrühe das Oxidationsmittel Thiosulfat umfasst und die L-Cystein enthaltende Fermenterbrühe bei einem pH Wert von pH 5 bis 9 mit einem stark sauren Ionenaustauscher in der H⁺-Form in Kontakt gebracht wird wobei ein pH-Wert von pH<5, bevorzugt pH<2 in der Fermenterbrühe auftritt und wobei das L-Cystein an den Ionenaustauscher bindet und das gebundene L-Cystein mittels eines Elutionsmittels von dem Ionenaustauscher entfernt wird.

2. Verfahren nach Anspruch 1, **dadurch gekennzeichnet, dass** als Elutionsmittel eine wässrige Salzsäure, eine wässrige Salzlösung, ein Amin, eine Base oder eine starke Base verwendet wird.

3. Verfahren nach einem der Ansprüche 1 oder2, **dadurch gekennzeichnet, dass** als Elutionsmittel zunächst 1N HCl zur Elution von L-Cystein und anschließend 2N HCl zur Elution von L-Cystin verwendet wird.

4. Verfahren nach einem der Ansprüche 1 bis 3, **dadurch gekennzeichnet, dass** die erhaltene Lösung zunächst aufkonzentriert wird, und anschließend aus der aufkonzentrierten Lösung L-Cystein-Hydrochlorid-Monohydrat auskristallisiert wird.

## Claims

1. Process for purifying an L-cysteine-containing fermenter broth, **characterized in that** the fermenter broth comprises the oxidizing agent thiosulfate and said L-cysteine-containing fermenter broth is contacted with a strongly acidic ion exchanger in the H⁺ form at a pH from pH 5 to 9, with the pH in the fermenter broth being pH<5, preferably pH<2, and said L-cysteine binding to said ion exchanger and the bound L-cysteine being removed from the ion exchanger by means of an eluant.

2. Process according to Claim 1, **characterized in that** the eluant used is an aqueous hydrochloric acid, an aqueous salt solution, an amine, a base or a strong base.

3. Process according to either of Claims 1 and 2, **characterized in that** the eluants used are firstly 1N HCl, for eluting L-cysteine, and then 2N HCl for eluting L-cystine.

4. Process according to any of Claims 1 to 3, **characterized in that** the solution obtained is first concentrated, and L-cysteine hydrochloride monohydrate is then crystallized from the concentrated solution.

## Revendications

1. Procédé pour la purification d'un bouillon de fermenteur contenant de la L-cystéine, **caractérisé en ce que** le bouillon de fermenteur comprend l'oxydant thiosulfate et on met en contact le bouillon de fermenteur contenant de la L-cystéine, à un pH de 5 à 9, avec un échangeur d'ions fortement acide sous la forme H⁺, dans lequel une valeur de pH de pH<5, de préférence pH<2 apparaît dans le bouillon de fermenteur et dans lequel la L-cystéine se fixe à l'échangeur d'ions et on sépare de l'échangeur d'ions la L-cystéine fixée, au moyen d'un éluant.

2. Procédé selon la revendication 1, **caractérisé en ce qu'**on utilise comme éluant un acide chlorhydrique aqueux, une solution aqueuse d'acide chlorhydrique, une amine, une base ou une base forte.

3. Procédé selon l'une quelconque des revendications 1 ou 2, **caractérisé en ce qu'**on utilise comme éluant d'abord HCl 1 N pour l'élution de la L-cystéine et ensuite HCl 2 N pour l'élution de la L-cystine.

4. Procédé selon l'une quelconque des revendications 1 à 3, **caractérisé en ce que** d'abord on concentre la solution obtenue, et ensuite on sépare de la solution concentrée le chlorhydrate de L-cystéine monohydraté, par cristallisation.
